(19) Europäisches Patentamt
European Patent Office
Office européen des brevets

(11) EP 1 865 961 B1

(12) **EUROPEAN PATENT SPECIFICATION**

(45) Date of publication and mention of the grant of the patent:
**15.02.2012 Bulletin 2012/07**

(21) Application number: **06739956.8**

(22) Date of filing: **29.03.2006**

(51) Int Cl.:
***A61K 31/522*** *(2006.01)* ***A61P 31/22*** *(2006.01)*

(86) International application number:
**PCT/US2006/011498**

(87) International publication number:
**WO 2006/105216 (05.10.2006 Gazette 2006/40)**

(54) **FAMCICLOVIR FOR THE TREATMENT OF RECURRENT HERPES LABIALIS USING A ONE-DAY TREATMENT**

FAMCICLOVIR ZUR BEHANDLUNG VON REZIDIVIERENDEM HERPES LABIALIS MITTELS EINMAL-TÄGLICH-BEHANDLUNG

FAMCICLOVIR POUR LE TRAITEMENT DE L'HERPES LABIAL RECURRENT PENDANT UNE PERIODE D'UN JOUR

(84) Designated Contracting States:
**AT BE BG CH CY CZ DE DK EE ES FI FR GB GR HU IE IS IT LI LT LU LV MC NL PL PT RO SE SI SK TR**

(30) Priority: **30.03.2005 US 666536 P**

(43) Date of publication of application:
**19.12.2007 Bulletin 2007/51**

(60) Divisional application:
**10179668.8 / 2 363 131**

(73) Proprietors:
- **Novartis AG**
  **4056 Basel (CH)**
- **Novartis Pharma GmbH**
  **1230 Wien (AT)**
  Designated Contracting States:
  **AT**

(72) Inventors:
- **BILLSTEIN, Stephan**
  **Franklin Lakes, NJ 07417 (US)**
- **CHARNAS, Robert**
  **Westlake Village, CA 91362 (US)**
- **SPRUANCE, Spotswood, L.**
  **Salt Lake City, UT 84103 (US)**

(74) Representative: **Crawley, Patrick Edward**
**Novartis AG**
**Corporate Intellectual Property**
**4002 Basel (CH)**

(56) References cited:
**US-A- 5 916 893**

- **SPRUANCE ET AL: "Treatment of herpes simplex labialis" HERPES, vol. 9, no. 3, 2002, - 2002 pages 64-69, XP009070057**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 865 961 B1

## Description

### Field of the Invention

This invention relates to the treatment of recurrent herpes labialis using a one-day treatment regimen and to the use of compounds in the preparation' of a medicament for use in a one-day treatment regimen of this condition.

### Background of the Invention

EP-A-141927 (Beecham Group p.l.c.) discloses penciclovir, the compound of formula (A):

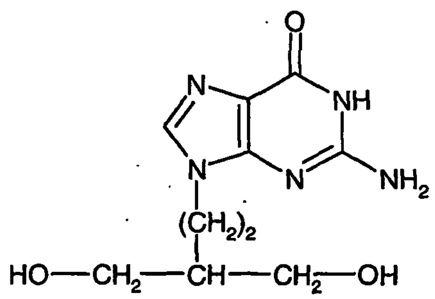

(A)

and salts, phosphate esters and acyl derivatives thereof, as antiviral agents. The sodium salt hydrate of penciclovir is disclosed in EP-A-216459 (Beecham Group p.l.c.). Penciclovir and its antiviral activity is also disclosed in Abstract P. V11-5, p.193 of Abstracts of 14th Int. Congress of Microbiology, Manchester, England, Sep. 7-13, 1986 (Boyd et. al.).

Orally active bioprecursors of the compound of formula (A) are of formula (B):

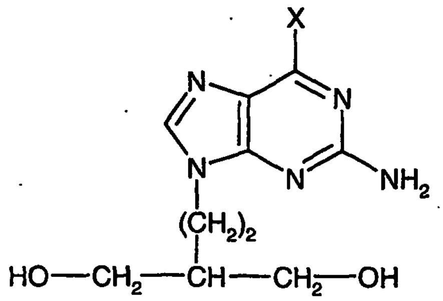

(B)

and salts and derivatives thereof as defined under formula (A), wherein X is $C_{1-6}$alkoxy, $NH_2$ or hydrogen. The compounds of formula (B) wherein X is $C_{1-6}$alkoxy or $NH_2$ are disclosed in EP-A-141927 and the compounds of formula (B), wherein X is hydrogen, disclosed in EP-A-182024 (Beecham Group p.l.c.) are preferred prodrugs. A particularly preferred example of a compound of formula (B) is that wherein X is hydrogen and wherein the two OH groups are in the form of the acetyl derivative, described in Example 2 of EP-A-182024, hereinafter referred to as famciclovir.

The compounds of formula (A) and (B) and salts and derivatives thereof have been described as useful in the treatment of infections caused by herpes viruses, such as herpes simplex type 1 (HSV-1), herpes simplex type 2 (HSV-2), varicella-zoster and Epstein-Barr viruses. Specifically, HSV-1 is the main cause of herpes labialis.

Recurrent herpes labialis is a common disease occurring in up to 40% of the adult population. It is caused mostly by HSV-1, which is usually acquired during childhood with a seroprevalence as high as 90% in persons over the age of 50 years. Although benign in most cases, herpes labialis may be associated with transient but real consequences such as significant irritation, pain, and discomfort in a social milieu. In susceptible persons, like neonates and immunocompromised patients, HSV can lead to significant morbidity.

Currently available topical or systemic treatment options consist of nucleoside analogues, such as acyclovir and penciclovir. Topical treatments containing either acyclovir or penciclovir reduce the duration of herpes labialis episodes with faster lesion healing and resolution of pain as compared to placebo. Typically they require multiple applications for several days and do not prevent the development of lesions (Jensen 2004).

Oral acyclovir is not approved for the treatment of orolabial herpes infection. However, patients treated with

acyclovir (200 mg or 400 mg five times a day for five days) have experienced a significant but limited reduction in time to healing of vesicular lesions and pain (Jensen 2004). None of the treatments increased the proportion of patients with aborted lesions. Valacyclovir was recently approved for the treatment of herpes labialis at a recommended dosage of 2,000 mg twice a day for one day. The efficacy of this treatment was evaluated in two randomized, double-blind, placebo-controlled studies in immunocompetent adult patients with more than three episodes during the previous year. The median time to healing of lesions was reduced by approximately one day with valacyclovir as compared to placebo (Spruance 2003). Famciclovir is approved for the treatment of recurrent orolabial HSV infection in HIV-infected patients. In a randomized, double-blind, dose ranging study, immunocompetent adult patients with a history of recurrent herpes labialis had a shorter time to healing and a reduction of the size of lesions when treated with famciclovir 500 mg three times a day for five days as compared to placebo (Spruance 1999).

However, there remains a need for therapy which alleviates or shortens the duration of symptoms associated with recurrent herpes labialis. The advantages of such treatment also serves to provide an effective and convenient therapy which not only may reduce the burden on healthcare budgets but increases treatment compliance.

## Summay of the Invention

The present invention is to the use of compounds of Formulae (A) and (B) as described herein, preferably famciclovir or penciclovir, for the treatment, of recurrent herpes labialis using a one-day treatment regimen.

## Detailed Description of the Invention

It has now been discovered that the above compounds are particularly effective in reducing the time to healing or duration of lesions of recurrent herpes labialis in immunocompetent patients when given as a high dose one-day treatment.

Accordingly, the present invention provides a method of treatment of Recurrent herpes labialis in humans, which method comprises the administration for a treatment period of one day to the human in need of such treatment, an effective amount of a compound of formula (A):

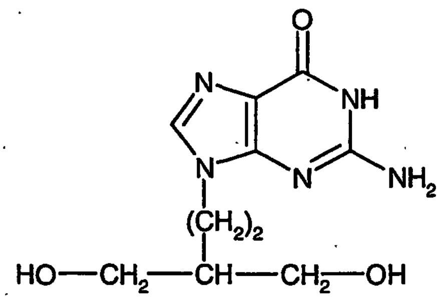

(A)

,

or a bioprecursor, or a pharmaceutically acceptable salt, phosphate ester and/or acyl derivative of either of the foregoing.

The term "acyl derivative" is used herein to include any derivative of the compounds of formula (A) in which one or more acyl groups are present. Such derivatives are included as bioprecursors of the compounds of formula (A) in addition to those derivatives which are *per se* biologically active.

The compound of formula (A) may be in one of the forms disclosed in EP-A-216459 (Beecham Group p.l.c.).

Examples of bioprecursors, pharmaceutically acceptable salts and derivatives are as described in the aforementioned European Patent references, the subject matter of which are incorporated herein by reference.

A particular compound of formula (B) of interest is 9-(4-acetoxy-3-acetoxymethylbut-1-yl)-2-aminopurine, known as famciclovir (FCV), the well-absorbed oral form of penciclovir (PCV).

The compound of formula (A), bioprecursors, salts and derivatives may be prepared as described in the aforementioned European Patent references.

The compound, in particular, famciclovir, may be administered by the oral route to humans and may be compounded in the form of syrup, tablets or capsule. When in the form of a tablet, any pharmaceutical carrier suitable for formulating such solid compositions may be used, e.g., magnesium stearate, starch, lactose, glucose, rice, flour and chalk. The compound may also be in the form of an ingestible capsule, e.g., of gelatin, to contain the compound, or in the form of a syrup, a solution or a suspension. Suitable liquid pharmaceutical carriers include ethyl alcohol, glycerine, saline and water to which flavouring or colouring agents may be added to form syrups. Sustained release formulations, for example tablets containing an enteric coating, are also envisaged.

For parenteral administration, fluid unit dose forms are prepared containing the compound and a sterile vehicle. The compound depending on the vehicle and the concentration, can be either suspended or dissolved. Parenteral solutions are normally prepared by dissolving the compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the compound is suspended in the vehicle instead of being dissolved and sterilised by exposure to ethylene oxide before suspending in the sterile vehicle. Advantageously, a surfactant or wetting agent is included in the composition to facilitate uniform distribution of the compound of the invention.

Preferred parenteral formulations include aqueous formulations using sterile water or normal saline, at a pH of around 7.4 or greater, in particular, containing penciclovir sodium salt hydrate.

As is common practice, the compositions will usually be accompanied by written or printed directions for use in the medical treatment concerned.

A suitable dosage unit might contain from 50-1,500 g of active ingredient, e.g., 250-1,000 mg. Such doses may be administered as a one-day treatment, such as 250 mg six times in one day, 500 mg three times in one day, 750 mg twice in one day or 1,500 mg once in one day or any suitable dosing scheme resulting in 1,500 mg dose in one day.

The treatment period is 1 day.

The short course treatment of the present invention is preferably carried out as soon as possible after the onset of the prodrome that precedes clinical signs of recurrent herpes labialis, usually within 24 hours, preferably within 12 hours, more preferably within one hour of the first prodromal symptom without any clinical signs of visible lesions.

The classic herpes labialis lesion progresses through stages involving erythema, papules, vesicles, ulcers, crusts, loss of crusts and loss of erythema. The most consistent clinical assessment for vesicular (classic) herpes labialis lesions is time of lesion healing following antiviral therapy.

"Prodrome" refers to focal itching, burning, tingling and/or pain at the site where cold sores have occurred in the past and felt by the study patient to be premonitory, , for him or her, of the onset of a cold sore.

"Erythema" refers to any redness, but without evidence of a more advanced stage. The presence of any physical sign of an episode indicates the end of the prodrome even if itching, pain, etc., associated with the prodrome continues.

"Papule" refers to any swelling or solid elevation of the skin without evidence of a more advanced stage.

"Vesicle" refers to any presence of a blister-like skin elevation of the skin in which fluid is visible through the stratum corneum, without evidence of a more advanced stage. Development of any evidence of vesiculation, ulceration or crusting defines lesions as vesicular, also referred to as "classical". When vesicles, ulcers and/or hard crusts are present together, then the stage of the vesicular (classical) lesion shall be described according to the predominant stage.

"Ulcer/soft crust" refers to the blister has collapsed or ruptured forming an ulcer. The floor of the ulcer may be moist or contain some soft cake-like exudate. When vesicles, ulcers and/or hard crusts are present together, then the stage of the (vesicular) classical lesion shall be described according to the predominant stage.

"Hard crust" refers to the drying of the ulcer has continued to form a noticeably hard, consolidated, unpliable mass, or a scab, an eschar. When vesicles, ulcers and/or hard crusts are present together, then the stage of the (vesicular) classical lesion shall be described according to the predominant stage.

"Residual abnormalities" refers to swelling, dry skin flakes and/or erythema that may be present after loss of the hard crust.

"Normal skin" refers to complete disappearance of all signs of the disease.

"Healed, (vesicular) classical lesions" refers to complete loss of crust. Residual abnormalities may still be present.

"Healed, aborted lesions" refers to complete disappearance of all signs of the disease (normal skin).

The present invention also provides the use of a compound of formula (A) or a bioprecursor, or a pharmaceutically acceptable salt, phosphate ester and/or acyl derivative of either of the foregoing, in the preparation of a medicament for use in a one day treatment regimen of herpes labialis and in particular in reducing the time to healing of herpes labialis lesions. Such treatment may be carried out in the manner as described herein.

The present invention further provides a pharmaceutical composition for use in a one day treatment regimen of recurrent herpes labialis, and in particular in reducing the time to healing of herpes labialis lesions, which comprises an effective amount of a compound of formula (A) or a bioprecursor, or a pharmaceutically acceptable salt, phosphate ester and/or acyl derivative of either of the foregoing, and a pharmaceutically acceptable carrier. Such compositions may be prepared in the manner as hereinafter described.

The compound of formula (A) and its prodrugs show a synergistic antiviral effect in conjunction with interferons; and treatment using combination products comprising these two components for sequential or concomitant administration, by the same or different routes, are therefore within the ambit of the present invention. Such products are described in EP-A-271270 (Beecham Group p.l.c.).

The following clinical data illustrate the invention.

Design

This study design is a parallel-group, double-blind, double-dummy, randomized placebo- controlled trial of patient initiated therapy in adult immunocompetent patients with recurrent herpes labialis.
Adult immunocompetent patients with a history of recurrent herpes labialis and at least three episodes in the previous 12 months are selected. These patients have a history of prodromal symptoms preceding at least 50% of their herpes labialis episodes and a history of vesicular lesions occurring in at least 50% of those episodes.
After screening, a total of 900 eligible patients are randomly assigned to one of three treatment groups and are dispensed study medication. The overall assignment ratio is 1:1:1. Patients are instructed to initiate treatment within one hour of the first prodromal symptom without any clinical signs of a recurrent episode of herpes labialis.
Each patient takes a total of nine capsules during the one-day study treatment as follows:

- Group 1, famciclovir 1500 mg daily (1500 mg po followed by placebo 12 hours later)
- Group 2, famciclovir 1500 mg daily (750 mg po b.i.d.) or
- Group 3, matching placebo capsules (po b.i.d.)..

After initiating therapy, patients are asked to return to the clinic within 24 hours for initial clinic assessment. Patients who miss the one-hour window or who have symptoms or signs of intraoral lesions or lesions within the nares are instructed to wait until the next recurrence of herpes labialis. Neither intraoral lesions nor lesions within the nares are treated within this protocol.
After the initial visit patients are required to return to the clinic once a day for three consecutive days and then every other day until lesion healing.
The maximum time in the study after randomization is 10 months.
Table 1 below summarizes the study design for herpes labialis treatment.

**Table 1. Study Design**

| **Pre-treatment period** | **Treatment period (1st clinical evaluation)** | **Continued Clinical Evaluation** | | | | |
|---|---|---|---|---|---|---|
| | Active Episode of Recurrent Herpes Labialis starting with prodrome | | | | | |
| Screening | Day 1 | Day 2 | Day 3 | Day 5 | Day 7 | F/U# |
| Randomization * | | | | | | |
| Visit 1 | Visit 2 | Visit 3 | Visit 4 | Visit 5 | Visit 6 | Every other day until lesion healing. |
| Dispense - Study Drug (SD) | SD- Start | SD none | SD none | SD none | SD none | SD none |
| ***Study medication dispensed.**<br># Patients with lesions followed until complete healing of the lesion occurs. | | | | | | |

Measurements

The criteria for evaluation include the primary efficacy variable and secondary efficacy variables. The primary efficacy variable is the investigator-assessed time to healing (re-epithelialization) of the non-aborted primary lesion complex, defined as the time from start of treatment until loss of crust (erythema may have been present). Secondary efficacy variables include time to healing of all non-aborted lesions (primary lesion complex and secondary lesions), time to healing of all lesions (non-aborted and aborted; latter assigned a time to healing of zero), and proportion of patients with aborted lesions.
Lesion healing for vesicular lesions is defined as loss of crust, although erythema may still be present. At each visit starting with Visit 2, the investigator makes assessments based on both the investigator examination of the current visit and patient diary entries since the previous visit. If healing is confirmed, the time of healing is defined as the earliest

time it was reported and continuously maintained in the diary. This convention applies to the assessment of both the primary lesion complex and all vesicular lesions.

The types of lesions measured are defined as follows:

"Primary lesion complex" refers to the first lesions that appear during the recurrent episode, additional lesions appearing on the same day as the first lesions, or lesions appearing later but within 1 cm of the previous lesions

"Secondary lesions" refer to lesions that appear more than 24 hours outside of the 1 cm vicinity of the primary lesion complex.

"Non-aborted lesions" refers to all lesions requiring re-epithelialization (i.e., lesions undergoing vesicular, ulcer, soft crust and/or hard crust formation)

"Aborted lesions" refers to lesions that did not evolve beyond the papule stage.

During lesion assessment, the investigator identifies patients whose herpes labialis recurrence did not progress beyond the papule stage based on the examination and diary entries. If this determination cannot be made due to missed visits and diary entries, the patient is assumed to have had classical (vesicular) lesions in the unobserved period.

Changes in the lesions are noted at each visit and the lesion stages recorded. Since more than one lesion stage can be present at any given time, but only one will be transcribed into the records, it is critical to select the lesion stage (a lesion stage may have several lesions in it) that best characterize the disease. The lesion stage identification should be the most biologically meaningful for the study while also practical for study personnel. Some lesion stages will be missed by the patient and/or the investigator because they have been too brief or occurred at night. For example, an apparent hard crust can temporarily revert to an ulcer following bathing.

When assessing the stage of the primary lesion complex, the following criteria are also determined:

(a) any presence of the next most advanced stage;
(b) the predominant (>50%) stage; or
(c) complete absence of or disappearance of a stage.

<u>Statistical analysis</u>

The primary efficacy analysis of this study compares the time to healing of the primary lesion complex in a single episode of herpes labialis for each of the active treatment regimens with placebo. The comparisons are presented using estimated median times to healing and their confidence intervals, as well as inferential analyses on the time to healing.

Inferential analyses of the primary outcome assesses the superiority of either of the famciclovir short-course regimens to placebo, as measured by the time to healing of the primary lesion complex. The overall type-one error rate is maintained at the 5% level for these tests.

Secondary analyses assess the famciclovir regimens, as compared to placebo, on their safety and tolerability; their efficacy in resolving pain and tenderness compared to placebo; their efficacy in preventing the outbreak of cold sores (vesicular (classical) lesions) by evaluating the proportion of patients with aborted lesions; and their efficacy as measured by the time to healing of all lesions (vesicular (classical) and aborted).

The data is summarized with respect to patient disposition, demographic and baseline characteristics, study medication, concomitant therapy, efficacy evaluation and safety evaluation. All continuous variables are summarized using descriptive statistics (mean, median, standard deviation, minimum and maximum). Categorical variables are summarized using frequency tables. Time-to-event variables are summarized using quartiles.

A full statistical analysis plan is produced prior to unblinding the study.

The Kaplan-Meier method is used to estimate the distribution of time to healing for each treatment group and the median time to healing. Estimated survival curves are plotted. The 95% confidence intervals for the median times to healing are constructed based on the first-order Taylor series approximation of their standard errors using the standard errors of the estimated survival functions. The 95% confidence intervals for between-treatment median differences are constructed using standard errors.

The overall distribution of the time to healing will be compared among treatment groups using the proportional hazards model, with treatment and center as the explanatory variables. The Efron approximate likelihood method will be used for resolution of ties. Confidence intervals for the hazard ratios will be provided.

The following two comparisons based on the proportional hazards model above are used for the primary efficacy analysis (for any active treatment T, $\theta_T$ stands for its log-hazard ratio compared to placebo):

(1) Famciclovir 1500 mg single dose vs. placebo
$H_{O1}$: $\theta_{famciclovir}$ 1500 mg = 0,

(2) Famciclovir 750 mg b.i.d, for one day vs. placebo

$H_{O2}$: $\theta_{famciclovir\ 750}$ mg b.i.d. 1 day = 0.

The modified Bonferroni procedure for multiple comparisons proposed by (Hochberg 1988) is used as follows to maintain the multiple (overall) 5% level of significance: if both comparisons are simultaneously significant at 5% level then the null hypotheses of both comparisons are rejected at the 5% level. Otherwise, each comparison will be separately tested at the significance level of 2.5%.

***Sensitivity and exploratory analysis***

The primary efficacy analysis will be repeated over the per-protocol population for sensitivity purposes. The assumption of proportional hazards for treatment regimens is assessed using log-log survival plots. Proportional treatment effects over time are expected based on data in the literature. However, if marked non-proportionality are found, such as when the treatment effects were larger in the first 2-3 days and smaller thereafter, presentation of the data by partitioning the time axis accordingly are considered.

Covariates and interactions are assessed by adding their respective terms separately into the primary efficacy analysis model: Covariates and interactions include: gender, age, treatment-by-gender interaction, treatment-by-center interaction, number of herpes labialis recurrences in the previous year (dichotomized), any other covariates may be added in the analysis plan.

Safety evaluation

The assessment of safety is based mainly on the frequency of adverse events. Adverse events are summarized by presenting, for each treatment group, the number and percentage of patients having any adverse event, having an adverse event in each body system and having each individual adverse event. Any other information collected (e.g., severity or relatedness to study medication) will be listed as appropriate.

Safety, intent-to-treat (ITT), and modified ITT populations

The safety and ITT populations include all randomized patients who are exposed to (take any) study medication. Using the above definitions, the safety and ITT populations comprise the same patients. For compatibility with the standard presentations, all safety summaries are referenced using the Safety population while appropriate efficacy summaries (for secondary parameters involving both vesicular and non-vesicular lesions) are referenced using the ITT population.

The modified ITT population includes all patients who develop herpes labialis vesicular lesions during the treated recurrence. The evaluable population used in sample size and power estimation includes all modified ITT patients who remain in the study until the time of healing can be determined.

Per-protocol (PP) population

This population includes all modified ITT patients without any major violations from study procedures. Deviations from the protocol that will be considered major violations include: taking the first dose of study medication more than one hour after the first prodromes; and taking antiviral medication other than the study medication between the day prior to the first dose of study medication to healing of primary lesion complex. For sensitivity and exploratory reasons, the primary efficacy analysis is repeated for the per-protocol population.

*Disposition*

The number of patients randomized and in each analysis population will be presented by treatment. For patients in the ITT population, the following information will be summarized: duration in study as measured from first dose of study medication to final visit, number of study visits, number of patients who discontinue from the study, reasons of discontinuation, and protocol deviations and violations.

***Demographics and background characteristics***

Descriptive statistics of patient demographics and other background characteristics at screening, as well as testing results of viral samples, are presented by treatment over the ITT population. Statistical comparisons among treatment groups on demographic variables are made, with p-values. For categorical variables (e.g., gender, race), the comparison are based on the Cochran-Mantel-Haenszel test stratified by center. For variables measured on a continuous

scale, (e.g., age), an analysis of variance (ANOVA) model is used with treatment and center as factors. These p-values are for descriptive purposes and will not be used to determine covariate inclusion in efficacy models.

Sample size and power considerations

The planned sample size of the study js 150 evaluable patients per treatment group. It is chosen to provide reliable estimates for treatment effects of two regimens of famciclovir (1,500 mg as a single dose or 750 mg b.i.d. for one day), as compared to that of placebo, based on the median time to healing of the primary vesicular lesion complex.

For illustrative purposes, the width of the confidence interval for the median time is anticipated. Using the definition of the confidence interval as the non-rejection region of hypothesis tests, the minimum size of median difference that will be detected as significant is found. This test on median difference is converted by parameterization to an equivalent hypothesis test for the hazard ratio.

Based on data in the literature, the median time to healing in recurrent herpes labialis for placebo is between 5 and 5.5 days. The healing process for a population of patients tends to exhibit acceleration consistent with the Weibull distribution with survival function of the form

$$S(t) = \exp(-\lambda t^{\gamma}), \text{ with } \gamma = 2.$$

For a comparison between one active treatment group with placebo, each with 150 patients, the estimates for power of the log-rank test are as follows. Note that under the distributional assumptions above, the tests on hazard rates are the same as those on the median healing times.

**Table 2. Interpretation of Hazard Ratio as Median Differences, and Approximate Power for the Log-Rank Test**

| Hazard ratio | Median time for Placebo (days) | Difference in median (days) # | Hazard ratio | Test size ($\alpha$) | Power* |
|---|---|---|---|---|---|
| 1.25 | 5 | 0.53 | 1.25 | 0.05 | 48% |
| | 5.5 | 0.58 | | 0.025 | 37% |
| 1.30 | 5 | 0.61 | 1.30 | 0.05 | 61% |
| | 5.5 | 0.68 | | 0.025 | 50% |
| 1.35 | 5 | 0.70 | 1.35 | 0.05 | 73% |
| | 5.5 | 0.77 | | 0.025 | 63% |
| 1.40 | 5 | 0.77 | 1.40 | 0.05 | 82% |
| | 5.5 | 0.85 | | 0.025 | 74% |
| 1.45 | 5 | 0.85 | 1.45 | 0.05 | 88% |
| | 5.5 | 0.93 | | 0.025 | 82% |

#Assuming Weibull distribution with shape parameter $\gamma = 2$
* From n-Query Advisor® 4.0

It can be seen that any difference in median of more than 1 day will be detected with at least 88% power. As a result, with a sample size of 150 patients in each group, it is anticipated with more than 88% probability that the confidence interval for the median survival time will be fit within one day around the estimated median.

Table 2 is used directly in assessing the power of testing the following two hypotheses based on the primary efficacy variable, time to healing of the primary lesion complex (for any active treatment T, $\theta_T$ stands for its log-hazard ratio compared to placebo):

(1) Famciclovir 1,500 mg single dose vs. placebo
$H_{O1}$: $\theta_{famciclovir}$ 1500 mg = 0,

(2) Famciclovir 750 mg b.i.d. (*bis in die* or twice a day) for one day vs. placebo
$H_{O2}$: $\theta_{famciclovir}$ 750 mg b.i.d.1 day = 0.

If any particular active treatment group has a hazard ratio of 1.45, the power to detect its difference from placebo following the Bonferroni-Hochberg procedure is not less than 82%.

Efficacy evaluation

Confidence intervals presented will be based on 95% coverage. All statistical tests are two-sided using $\alpha = 0.05$.

Primary efficacy variable

Variable 1, the primary efficacy variable, is the investigator-assessed time to healing of the primary lesion complex, defined as the time from start of treatment until loss of crust (for vesicular (classical) lesions progressing through the vesicle/ulcer stage only, erythema is allowed).

The primary efficacy variable, investigator-assessed time to healing of the primary lesion complex, is analyzed over the modified ITT-population in two approaches: the primary estimation is based on the Kaplan-Meier method, and statistical testing is based on the proportional hazards model.

Secondary efficacy variable

Secondary efficacy variables include variables 2-7. Variable 2 is the time to healing (lost of crust) of the primary lesion complex (non-vesicular lesions will be assigned a time of zero). Variable 3 is the time to healing (loss of crust) of all vesicular lesions (non-vesicular lesions will be assigned a time of zero). Variable 4 is the time to return to normal skin for all lesions (no erythema). Variable 5 is the proportion of patients with aborted lesions (i.e., non-vesicular). Variable 6 is the percentage of patients with lesion tenderness and pain. Variable 7 is the duration of lesion tenderness and pain, defined from the onset to the time of disappearance:

Analyses of secondary efficacy variables is performed on the nominal $\alpha$-level of 0.05, without adjusting for multiplicity. The time-to-event variables, variables 2, 3, 4 and 7 listed above are analyzed using a proportional hazards model with treatment and center as explanatory variables, for patients over the ITT population. The Efron approximate livelihood method is used for resolution of ties. Each famciclovir regimen is compared against placebo. The 95% confidence intervals for the hazard ratios and treatment-specific median times is provided. Covariates and interactions listed for the primary efficacy variable are assessed separately by adding their terms into the proportional hazards model. The efficacy outcomes measured as proportions, variables 5 and 6, are compared between each famciclovir, treatment group and placebo using the Cochran-Mantel-Haenszel (CMH) test, stratified by study center.

The primary and key secondary efficacy results are shown in Table 3.

**Table 3. Primary and key secondary efficacy results (Study 2403)**

| | **Famciclovir 750 mg b.i.d.** | **Hazard ratio** | ***p*-value** | **Famciclovir 1500 mg q.d. (once daily)** | **Hazard ratio** | ***p*-value** | **Placebo** |
|---|---|---|---|---|---|---|---|
| **Time to healing of primary lesion complex (days)[1]** | | | | | | | |
| Modified ITT | 4.0 (3.8, 4.8) | 2.05 | <0.001 | 4.4 (3.9, 5.0) | 1.64 | <0.001 | 6.2 (5.7, 7.0) |
| Per Protocol | 4.0 (3.8, 4.8) | 2.06 | <0.001 | 4.4 (3.8, 5.0) | 1.71 | <0.001 | 6.1 (5.7, 7.2) |
| **Time to healing of all non-aborted (primary and secondary) lesions (days)[1]** | | | | | | | |
| Modified ITT | 4.1 (3.8, 5.0) | 2.06 | <0.001 | 4.5 (4.0, 5.0) | 1.71 | <0.001 | 6.6 (5.9, 7.3) |
| **Time to healing of all (non-aborted and aborted) lesions (days)[1,2]** | | | | | | | |
| ITT | 3.0 (2.4, 3.5) | 1.39 | 0.001 | 2.9 (2.4, 3.5) | 1.34 | 0.005 | 4.2 (3.0, 5.4) |
| **Proportion of patients with aborted lesions** | | | | | | | |
| ITT | 29% | | NS[3] | 33% | | NS[3] | 34% |

[1]Median time and 95% confidence interval around the median time are shown
[2]Aborted lesions were assigned a time to healing of zero
[3]NS = not significant

For the modified ITT population, both famciclovir 750 mg b.i.d. for one day and famciclovir 1,500 mg as a single dose are superior to placebo in reducing investigator-assessed time to healing of the primary lesion complex ($p<0.001$).

The famciclovir 750 mg and famciclovir 1,500 mg-placebo hazard ratios for time to healing were 2.05 and 1.64, respectively. The estimated median times to healing of the primary lesion complex were 4.0, 4.4 and 6.2 days for famciclovir 750 mg, famciclovir 1500 mg and placebo, respectively. Similar results are obtained when the analysis is conducted for the PP population. The results of time to healing of all non-aborted (primary and secondary) lesions and all (non-aborted and aborted) lesions are consistent with the primary efficacy variable (Table 3). There is no difference in the proportion of patients with aborted lesions (ranging from 29-34%) for any of the treatment arms. No significant differences are found between the two famciclovir treatment groups for any of the efficacy parameters tested.

Discussion

Time to healing of primary lesions (those developing on the first day) was significantly faster for FCV 1,500 mg once and 750 mg twice for one day compared with placebo, with median times of 4.4 and 4.0 days vs 6.2 days, respectively (p<.001). Similarly, time to healing of all lesions (primary and secondary combined) was reduced in the two FCV groups compared with the placebo group, with median times of 4.5 and 4.1 days vs 6.6 days, respectively ($p$<.001). Time to healing of lesions was similar in both FCV regimens. There were no differences across the groups with regard to the proportion of patients with aborted episodes. High-dose FCV was well tolerated and as safe as placebo.

If taken within one hour of the onset of the prodrome that precedes clinical signs of recurrent herpes labialis, one-day treatment with famciclovir either 750 mg b.i.d. or 1,500 mg q.d. significantly reduced the time to healing of lesions as compared to placebo. One day treatment with famciclovir for recurrent herpes labialis in immunocompetent patients is a, safe, well-tolerated and effective treatment which is superior to placebo in reducing time to healing of non-aborted primary lesions, non-aborted primary and secondary lesions, and all (non-aborted and aborted) lesions of recurrent herpes labialis.

## Claims

1. The compound 9-(4-acetoxy-3-acetoxymethylbut-1-yl)-2-aminopurine (Famciclovir), or a pharmaceutically acceptable salt thereof for use in treating recurrent herpes labialis in a human in need thereof, wherein said compound is to be administered for a period of one day.

2. The compound for use according to claim 1, wherein the one day treatment is commenced within one hour of onset of prodrome.

3. The compound for use according to claim 1, wherein the one day treatment is commenced within 12 hours of onset of prodrome.

4. The compound for use according to claim 1, wherein the one day treatment is commenced within 24 hours of onset of prodrome.

5. The compound for use according to claims 1 to 4, wherein the human in need is immunocompetent with recerrunt herpes labialis.

6. The compound for use according to claims 1 to 5, wherein said compound is to be administered at a dose of 1,500 mg for a period of one day.

7. The compound for use according to claims 1 to 5, wherein said compound is to be administered at a dose of 250 mg six times in one day.

8. The compound for use according to claims 1 to 5, wherein said compound is to be administered at a dose of 500 mg three times in one day.

9. The compound for use according to claims 1 to 5, wherein said compound is to be administered at a dose of 750 mg twice in one day.

10. The compound for use according to claims 1 to 5, wherein said compound is to be administered orally.

11. The compound for use according to claims 1 to 5, wherein said compound is to be administered parentally.

**Patentansprüche**

1. Die Verbindung 9-(4-Acetoxy-3-acetoxymethylbut-1-yl)-2-aminopurin (Famciclovir) oder ein pharmazeutisch akzeptables Salz hiervon zur Verwendung bei der Behandlung von rezidivierendem Herpes simplex der Lippen bei einem Menschen, der dies benötigt, wobei die Verbindung für eine Dauer von einem Tag zu verabreichen ist.

2. Die Verbindung zur Verwendung nach Anspruch 1, wobei mit der eintägigen Behandlung innerhalb einer Stunde nach Einsetzen einer Prodromalerscheinung begonnen wird.

3. Die Verbindung zur Verwendung nach Anspruch 1, wobei mit der eintägigen Behandlung innerhalb von 12 Stunden nach Einsetzen einer Prodromalerscheinung begonnen wird.

4. Die Verbindung zur Verwendung nach Anspruch 1, wobei mit der eintägigen Behandlung innerhalb von 24 Stunden nach Einsetzen einer Prodromalerscheinung begonnen wird.

5. Die Verbindung zur Verwendung nach einem der Ansprüche 1 bis 4, wobei der bedürftige Mensch mit rezidivierendem Herpes simplex der Lippen immunokompetent ist.

6. Die Verbindung zur Verwendung nach einem der Ansprüche 1 bis 5, wobei die Verbindung in einer Dosis von 1500 mg während eines Zeitraums von einem Tag zu verabreichen ist.

7. Die Verbindung zur Verwendung nach einem der Ansprüche 1 bis 5, wobei die Verbindung in einer Dosis von 250 mg sechsmal innerhalb eines Tages zu verabreichen ist.

8. Die Verbindung zur Verwendung nach einem der Ansprüche 1 bis 5, wobei die Verbindung in einer Dosis von 500 mg dreimal innerhalb eines Tages zu verabreichen ist.

9. Die Verbindung zur Verwendung nach einem der Ansprüche 1 bis 5, wobei die Verbindung in einer Dosis von 750 mg zweimal innerhalb eines Tages zu verabreichen ist.

10. Die Verbindung zur Verwendung nach einem der Ansprüche 1 bis 5, wobei die Verbindung oral zu verabreichen ist.

11. Die Verbindung zur Verwendung nach einem der Ansprüche 1 bis 5, wobei die Verbindung parenteral zu verabreichen ist.

**Revendications**

1. Composé 9-(4-acétoxy-3-acétoxyméthylbut-1-yl)-2-aminopurine (famciclovir), ou sel pharmaceutiquement acceptable de celui-ci, destiné à être utilisé dans le traitement de l'herpès des lèvres récurrent chez un être humain chez lequel cela est nécessaire, ledit composé devant être administré pendant une période d'une journée.

2. Composé destiné à être utilisé selon la revendication 1, où le traitement d'une journée est commencé dans l'heure qui suit le début du prodrome.

3. Composé destiné à être utilisé selon la revendication 1, où le traitement d'une journée est commencé dans les 12 heures qui suivent le début du prodrome.

4. Composé destiné à être utilisé selon la revendication 1, où le traitement d'une journée est commencé dans les 24 heures qui suivent le début du prodrome.

5. Composé destiné à être utilisé selon les revendications 1 à 4, où l'être humain chez lequel cela est nécessaire est immunocompétent avec un herpès des lèvres récurrent.

6. Composé destiné à être utilisé selon les revendications 1 à 5, ledit composé devant être administré à raison d'une dose de 1.500 mg sur une période d'une journée.

7. Composé destiné à être utilisé selon les revendications 1 à 5, ledit composé devant être administré à raison d'une

dose de 250 mg six fois dans une journée.

**8.** Composé destiné à être utilisé selon les revendications 1 à 5, ledit composé devant être administré à raison d'une dose de 500 mg trois fois dans une journée.

**9.** Composé destiné à être utilisé selon les revendications 1 à 5, ledit composé devant être administré à raison d'une dose de 750 mg deux fois dans une journée.

**10.** Composé destiné à être utilisé selon les revendications 1 à 5, ledit composé devant être administré par voie orale.

**11.** Composé destiné à être utilisé selon les revendications 1 à 5, ledit composé devant être administré par voie parentérale.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 141927 A
- EP 216459 A
- EP 182024 A
- EP 271270 A


### Non-patent literature cited in the description


- **Boyd.** Abstract P. V11-5. *Abstracts of 14th Int. Congress of Microbiology,* 07 September 1986, 193